# DEMANDE DE BREVET EUROPEEN

(11) **EP 0 601 930 A1**
(43) Date de publication de la demande: **15.06.1994**
(21) Numéro de dépôt: 93402967.9
(22) Date de dépôt: 09.12.1993
(51) Int. Cl.: C07D 263/32, A61K 31/42

(54) **Nouveaux dérivés de 4-méthyl-1,3-oxazole, leur procédé de préparation et les compositions pharmaceutiques qui les contiennent**

(30) Priorité: 11.12.1992 FR 9214912
(71) Demandeur: ADIR ET COMPAGNIE, F-92415 Courbevoie Cédex (FR)
(72) Inventeur: de Nanteuil, Guillaume, F-92150 Suresnes (FR); Vincent, Michel, F-92220 Bagneux (FR); Lila, Christine, F-78220 Viroflay (FR); Bonnet, Jacqueline, F-75013 Paris (FR); Fradin, Armel, F-92200 Neuilly Sur Seine (FR)

(57) **Abrégé**

Composé de formule (I) : 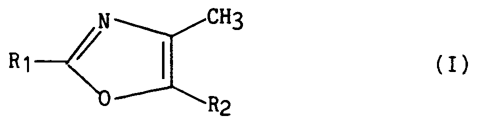
dans laquelle :
- R₁: représente un groupement 1-adamantyl, dicyclopropylméthyle, cycloalkyle (C₃-C₆) substitué ou non, bicyclo[2.2.2]oct-1-yl substitué ou non,
- R₂: représente un groupement

. 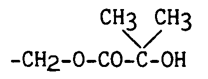 ou
. 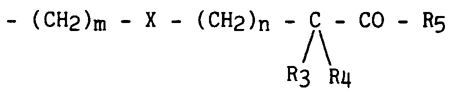 dans lequel :
   - m: représente 1, 2 ou 3,
   - X: représente un atome d'oxygène, de soufre ou un groupement N-R,
   - R₃ ou R₄,: identiques ou différents, représentent un atome d'hydrogène, un groupement alkyle (C₁-C₆), trifluorométhyle, ou 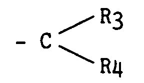 forme un radical cycloalkyle (C₃-C₆),
   - n: représente 0, 1 ou 2,
   - R₅: représente un groupement hydroxy, alkoxy (C₁-C₆), amino substitué ou non ou - O - CH₂ - CO - NRR'

Médicaments.

## Description

La présente invention concerne de nouveaux dérivés de 4-méthyl-1,3-oxazole, leur procédé de préparation et les compositions pharmaceutiques qui les contiennent.

Ces composés, outre le fait qu'ils soient nouveaux, possèdent des propriétés pharmacologiques qui les rendent utilisables dans le traitement de l'arthrite et de pathologies inflammatoires.

Au cours de la réaction inflammatoire, d'importantes modifications se produisent dans la synthèse d'un groupe de protéines plasmatiques appelées les protéines de la phase aiguë. Certaines de ces protéines - dont le fibrinogène, la protéine C réactive, l'haptoglobine - sont augmentées au cours de la réaction de la phase aiguë, alors que d'autres comme l'albumine et la transférrine sont diminuées. L'altération de ces protéines, en particulier du fibrinogène, est à l'origine des modifications de la viscosité plasmatique et de l'augmentation de la vitesse de sédimentation observées dans l'inflammation. En raison de leur corrélation avec les paramètres cliniques au cours de l'évolution et des rémissions thérapeutiques observées dans la polyarthrite rhumatoïde, certaines de ces protéines de la phase aiguë ont été utilisées comme critère d'évaluation de la maladie (Mallya RK & Coll., J. Rheumatol., 1982, 9, 224-8 ; Thompson PW & Coll., Arthritis Rheum 1987, 30, 618-23). Elles sont sous la dépendance de certaines cytokines, en particulier l'Il₁ et l'Il₆, reconnues pour jouer un rôle important dans la pathologie arthritique (Gauldie J & Coll., Cytokines and acute phase protein expression. In : Cytokines and Inflammation. Edited by ES Kimball. CRC Press, 1991, p 275-305).

En pharmacologie animale, les modifications des protéines de la phase aiguë ont été étudiées en particulier chez le rat au cours de la phase inflammatoire aiguë faisant suite à l'injection d'adjuvant complet (Lewis EJ & Coll., J. Pharmacol Meth 1989, 21, 183-94).

Un certain nombre de dérivés de 1,3-oxazole ont été décrits dans la littérature. C'est le cas, en particulier, des composés décrits dans le brevet EP 220573.

Plus spécifiquement, la présente invention concerne les composés de formule (I) 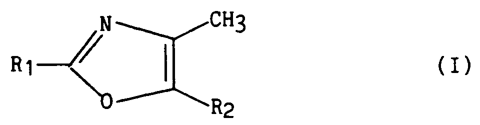
dans laquelle :
- R₁: représente un groupement 1-adamantyl, dicyclopropylméthyle, cycloalkyle (C₃-C₆) (substitué ou non par un atome d'halogène, un groupement hydroxy, alkoxy (C₁-C₆) linéaire ou ramifié) ou bicyclo[2.2.2]oct-1-yl (substitué ou non en position 4 par un atome d'halogène, un groupement alkoxy (C₁-C₆) linéaire ou ramifié ou hydroxy),
- R₂: représente un groupement
. 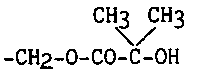 ou
. 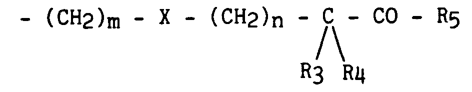
dans lequel :
m représente 1, 2 ou 3,
X représente un atome d'oxygène, de soufre ou un groupement N-R (dans lequel R est un atome d'hydrogène ou un groupement alkyle (C₁-C₆) linéaire ou ramifié),
R₃ ou R₄, identiques ou différents, représentent un atome d'hydrogène, un groupement alkyle (C₁-C₆) linéaire ou ramifié, trifluorométhyle,
ou 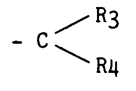 forme un radical cycloalkyle (C₃-C₆),
n représente 0, 1 ou 2,
R₅ représente un groupement hydroxy, alkoxy (C₁-C₆) linéaire ou ramifié, amino (substitué ou non par un ou deux groupements alkyle (C₁-C₆) linéaire ou ramifié),
- O - CH₂ - CO - NRR' (tel que R et R' représentent un groupement alkyle (C₁-C₆) linéaire ou ramifié, ou forment avec l'atome d'azote qui les portent un hétérocycle à 5 ou 6 chaînons),

leurs énantiomères, diastéréoisomères ou épimères ainsi que leurs sels d'addition à un acide ou à une base pharmaceutiquement acceptable.

Parmi les acides pharmaceutiquement acceptables, on peut citer à titre non limitatif les acides chlorhydrique, sulfurique, tartrique, maléique, fumarique, méthane sulfonique, camphorique, etc...

Parmi les bases pharmaceutiquement acceptables, on peut citer à titre non limitatif l'hydroxyde de sodium, l'hydroxyde de potassium, la tertbutylamine, la diéthylamine, l'éthylènediamine...

L'invention s'étend également au procédé de préparation des composés de formule (I) caractérisé en ce que l'on utilise comme produit de départ un acide de formule (II) :

R₁ - CO₂H (II)

dans laquelle R₁ a la même signification que dans la formule (I),
que l'on fait réagir sur le 2-chloroacétoacétate d'éthyle, pour conduire au composé de formule (III) : 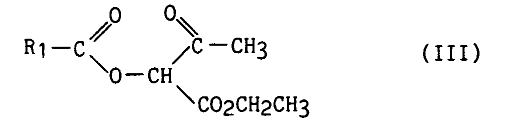
dans laquelle R₁ a la même signification que dans la formule (I),
qui subit l'action du formamide en milieu acide, pour conduire au composé de formule (IV) : 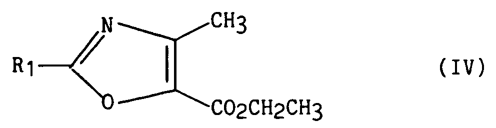
dans laquelle R₁ a la même signification que dans la formule (I),
que l'on transforme :
en composé de formule (V) par réduction en présence d'hydrure de lithium aluminium, 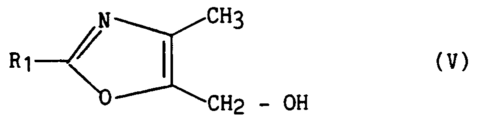
dans laquelle R₁ a la même signification que dans la formule (I),
sur lequel on fait réagir :
soit,
en milieu anhydre, l'acétone en présence d'hydroxyde de sodium séché et de chloroforme,
pour conduire au composé de formule (I/a), cas particulier des composés de formule (I) : 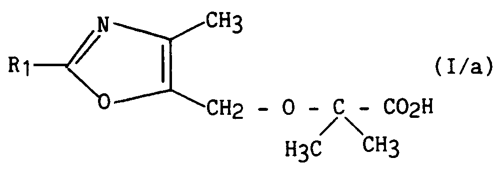
dans laquelle R₁ a la même signification que dans la formule (I), dont on transforme, si on le souhaite, la fonction acide en fonction ester ou amide selon des techniques classiques de la chimie organique,
soit,
le chlorure de thionyle, pour conduire au composé de formule (VI) : 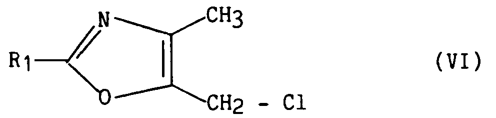
dans laquelle R₁ a la même signification que dans la formule (I),
que l'on peut faire réagir, si on le souhaite, avec le 2-hydroxyisobutyrate d'éthyle, en présence d'hydrure de sodium en milieu diméthylformamide non anhydre, pour conduire au composé de formule (I/b) 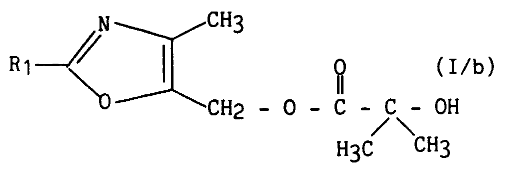
dans laquelle R₁ a la même signification que dans la formule (I), composé de formule (VI) qui peut subir des réactions classiques de la chimie organique conduisant au composé de formule (VII) : 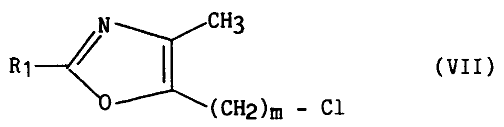
dans laquelle R₁ et m ont la même signification que dans la formule (I), que l'on fait réagir :
a avec un composé de formule (VIII), dans le DMF, en milieu anhydre : 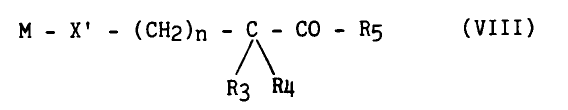 dans laquelle M représente un métal alcalin, n, R₃, R₄ et R₅ sont tels que définis dans la formule (I) et X' représente un atome de soufre ou d'oxygène,
   pour conduire au composé de formule (I/c), cas particulier des composés de formule (I), 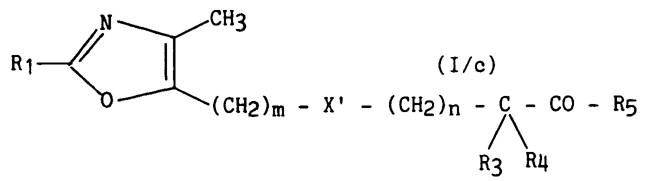 dans laquelle R₁, R₃, R₄, R₅, X', m et n sont tels que définis précédemment,
   dont on transforme, si on le souhaite, R₅ lorsqu'il représente un groupement hydroxy en groupement amino ou ester correspondant selon les techniques classiques de la chimie organique,
b avec un amino-ester de formule (IX) : 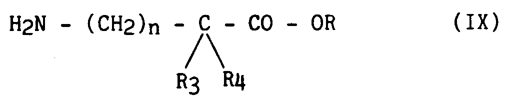 dans laquelle R représente un groupement alkyle, n, R₃ et R₄ ont la même signification que dans la formule (I), pour conduire au composé de formule( I/d), cas particulier des composés de formule (I) : 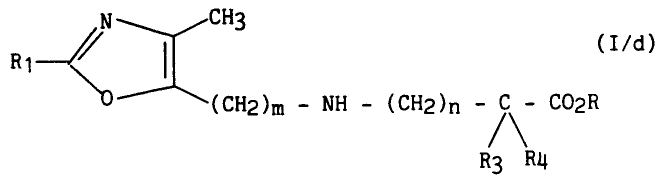 dans laquelle R₁, R₃, R₄, R, m et n ont la même signification que précédemment,
   dont on transforme, si on le souhaite, la fonction ester en fonction acide puis en fonction amide correspondante selon des techniques classiques de la chimie organique et la fonction amine secondaire en fonction amine tertiaire par alkylation,

composés de formule (I/a), (I/b), (I/c) ou (I/d) que l'on purifie, le cas échéant, selon une technique classique de purification dont on sépare, si on le souhaite, les isomères selon des techniques classiques de séparation, et que l'on transforme éventuellement en leurs sels d'addition à un acide ou à une base pharmaceutiquement acceptable.

Ces nouveaux dérivés de 4-méthyl-1,3-oxazole présentent des propriétés pharmacologiques très intéressantes. Ils diminuent les effets d'une injection d'adjuvant de Freund chez le rat tant au niveau des protéines plasmatiques de la phase aiguë (albumine) que de l'oedème local. Cet effet signale une activité antiinflammatoire des composés de l'invention.

L'invention s'étend aussi aux compositions pharmaceutiques renfermant comme principe actif au moins un composé de formule (I) ou ses isomères optiques avec un ou plusieurs excipients inertes, non toxiques et appropriés. Les compositions pharmaceutiques ainsi obtenues pourront être présentées sous diverses formes, les plus avantageuses étant les comprimés, les dragées, les gélules, suppositoires, suspensions buvables, les formes transdermiques (gel, patch), etc...

La posologie utile est adaptable selon la nature et la sévérité de l'affection, la voie d'administration ainsi que selon l'âge et le poids du patient. Cette posologie unitaire varie de 0,02 g à 2 g par jour en une ou plusieurs prises.

Les exemples suivants illustrent l'invention mais ne la limitent en aucune façon.
Les produits de départ utilisés sont des produits de départ connus ou préparés selon des modes opératoires connus.

### EXEMPLE 1 : Acide 2-méthyl-2-{[2-(adamant-1-yl)-4-méthyl-1,3-oxazol-5-yl] méthoxy}propionique, sel de sodium

### Stade A : 2-[(Adamant-1-yl)carbonyloxy]acétoacétate d'éthyle

A 100 mmoles de carbonate de sodium placées dans 160 ml de DMF sont ajoutées 200 mmoles d'acide 1-adamantane carboxylique. L'ensemble est chauffé à 80°C puis 200 mmoles de 2-chloroacétoacétate d'éthyle en solution dans 40 ml de DMF sont ajoutées. La température et l'agitation sont maintenues trois heures puis l'ensemble est abandonné 10 heures à température ambiante. Après évaporation du DMF, le résidu est repris par 300 ml d'eau et 300 ml d'éther. Après extraction, séchage et évaporation, on obtient le produit attendu sous forme d'huile.

### Stade B : 2-(Adamant-1-yl)-4-méthyl-5-éthoxycarbonyl-1,3-oxazole

A 27,7 ml de formamide anhydre, sont ajoutées, goutte à goutte, à 10°C, 3,8 ml d'acide sulfurique concentré puis 33 mmoles du produit obtenu au stade précédent. L'ensemble est chauffé 2 heures à 140°C. Après refroidissement à 10°C, 140 ml d'eau et 100 ml d'éther sont ajoutés. Après extraction, lavage de la phase organique par de l'acide sulfurique N/10, séchage et évaporation, le produit attendu est obtenu après purification sur colonne de silice en utilisant comme éluant un mélange dichlorométhane/acétate d'éthyle (95/5).

| Microanalyse élémentaire: | | | |
|---|---|---|---|
| | C % | H % | N % |
| calculé | 70,56 | 8,01 | 4,84 |
| trouvé | 70,59 | 7,78 | 5,18 |

### Stade C : 2-(Adamant-1-yl)-4-méthyl-5-hydroxyméthyl-1,3-oxazole

30 mmoles d'hydrure de lithium aluminium sont placées, sous atmosphère d'azote, dans 40 ml de THF, à 0°C. 23 mmoles du composé obtenu au stade précédent sont ajoutées à ce mélange et l'ensemble est laissé une heure à 0°C puis 2 heures à température ambiante. 7,5 ml d'isopropanol et 4,5 ml d'une solution saturée de chlorure de sodium sont alors ajoutés puis l'ensemble est abandonné 10 heures à température ambiante. Après filtration du précipité, le filtrat est évaporé et le résidu repris par 50 ml d'eau et 150 ml d'éther. Après extraction, lavage, séchage et évaporation, le produit attendu est obtenu sous forme de cristaux blancs.
Point de fusion : 134°C

| Microanalyse élémentaire: | | | |
|---|---|---|---|
| | C % | H % | N % |
| calculé | 72,84 | 8,56 | 5,66 |
| trouvé | 72,72 | 8,58 | 5,70 |

### Stade D : Acide 2-méthyl-2-{[2-(adamant-1-yl)-4-méthyl-1,3-oxazol-5-yl] méthoxy}propionique, sel de sodium

18 mmoles du composé obtenu au stade précédent sont placées dans 27 ml d'acétone anhydre. 92 mmoles d'hydroxyde de sodium sec et en poudre sont alors ajoutées. L'ensemble est porté au reflux de l'acétone jusqu'à coloration rouge. 24 mmoles de chloroforme dans 5,5 ml d'acétone sont ajoutées et le milieu porté 4 heures au reflux puis 10 heures à température ambiante. Après évaporation du solvant, le résidu est repris par 200 ml d'eau et lavé par 200 ml d'éther. La phase aqueuse est alors acidifiée par HCl 2N jusqu'à pH = 2. Le précipité est filtré, séché et lavé et est transformé en sel de sodium correspondant.

| Microanalyse élémentaire: | | | |
|---|---|---|---|
| | C % | H % | N % |
| calculé | 64,21 | 7,37 | 3,94 |
| trouvé | 64,02 | 7,35 | 3,87 |

### EXEMPLE 2: 2-Méthyl-2-{[2-(dicylopropylméthyl)-4-méthyl-1,3-oxazol-5-y] méthoxy}propionate d'éthyle

Les stades A, B et C sont identiques aux stades A, B et C de l'exemple 1 l'acide 1-adamantane carboxylique étant remplacé au stade A par l'acide dicyclopropylméthylcarboxylique.

### Stade D : 2-(1-dicyclopropylméthyl)-4-méthyl-5-chlorométhyl-1,3-oxazole

22 mmoles du composé obtenu au stade précédent dans 50 ml de dichlorométhane sont refroidies à 5°C. 3,25 ml de chlorure de thionyle sont alors ajoutés goutte à goutte. L'ensemble est porté 2 heures au reflux. Le produit attendu est alors obtenu sous forme d'huile après évaporation et séchage.

### Stade E : 2-Méthyl-2-{[2-(dicylopropylméthyl)-4-méthyl-1,3-oxazol-5-yl] méthoxy}propionate d'éthyle

51 mmoles d'hydrure de sodium sont placées dans 40 ml de DMF anhydre sous atmosphère d'azote. 51 mmoles de 2-hydroxy-isobutyrate d'éthyle dans 20 ml de DMF sont alors ajoutées et l'ensemble est abandonné une heure à température ambiante. Après refroidissement dans un bain de glace 36 mmoles du composé obtenu au stade précédent en solution dans 20 ml de DMF sont ajoutées. L'ensemble est agité une nuit à température ambiante. A -5°C, 20 ml d'une solution saturée de chlorure d'ammonium sont ajoutés. Après évaporation du DMF, reprise par 150 ml d'eau, extraction à l'acétate d'éthyle, lavage et séchage, le produit attendu est obtenu sous forme d'huile après évaporation et purification par chromatographie sur colonne de silice en utilisant comme éluant un mélange pentane/acétate d'éthyle (85/15).

| Microanalyse élémentaire: | | | |
|---|---|---|---|
| | C % | H % | N % |
| calculé | 67,26 | 8,47 | 4,36 |
| trouvé | 67,42 | 8,48 | 4,70 |

### EXEMPLE 3: Acide 2-méthyl-2-{[2-(dicyclopropylméthyl)-4-méthyl-1,3-oxazol-5-yl]méthoxy}propionique, sel de sodium

A une solution contenant 8 mmoles du produit obtenu au stade précédent dans 60 ml d'éthanol, on ajoute 8 mmoles de soude en pastilles puis 4 ml d'eau. L'ensemble est porté 3 heures au reflux. Après évaporation, reprise du résidu par de l'eau, extraction par de l'éther, acidification par de l'acide chlorhydrique 4N, le produit attendu est obtenu après filtration et séchage et est transformé en sel de sodium correspondant.

| Microanalyse élémentaire: | | | |
|---|---|---|---|
| | C % | H % | N % |
| calculé | 60,94 | 7,03 | 4,44 |
| trouvé | 60,99 | 7,02 | 4,79 |

Les exemples 4 et 6 ont été synthétisés selon le même mode opératoire que celui décrit pour l'exemple 2.

Les exemples 5 et 7 ont été synthétisés selon le même mode opératoire que celui décrit pour l'exemple 3.

### EXEMPLE 4: 2-Méthyl-2-{[2-(4-méthoxycyclohexyl)-4-méthyl-1,3-oxazol-5-yl] méthoxy}propionate d'éthyle

### EXEMPLE 5: Acide 2-méthyl-2-{[2-(4-méthoxycyclohexyl)-4-méthyl-1,3-oxazol-5-yl]méthoxy}propionique, sel de sodium Spectre de masse : FAB : [M+H]⁺ : m/z = 334

### EXEMPLE 6: 2-Méthyl-2-{[2-(bicyclo[2.2.2]oct-1-yl)-4-méthyl-1,3-oxazol-5-yl]méthoxy}propionate d'éthyle

### EXEMPLE 7: Acide 2-méthyl-2-{[2-(bicyclo[2.2.2]oct-1-yl)-4-méthyl-1,3-oxazol-5-yl]méthoxy}propionique, sel de sodium

### EXEMPLE 8: 2-(Adamant-1-yl)-4-méthyl-5-(2-hydroxyisobutyroxyméthyl)-1,3-oxazole

Les stades A à D de cet exemple sont réalisés selon les mêmes procédés que ceux décrits dans les stades A à D de l'exemple 2.

### Stade E : 2-(Adamant-1-yl)-4-méthyl-5-(2-hydroxyisobutyroxyméthyl)-1,3-oxazole

29 mmoles d'hydrure de sodium sont placées dans 20 ml de DMF. A 0°C, 29 mmoles de 2-hydroxyisobutyrate d'éthyle en solution dans 10 ml de DMF non anhydre sont ajoutées et l'ensemble est laissé une heure à température ambiante. On coule goutte à goutte, à 0°C, 21 mmoles du produit obtenu au stade précédent et l'agitation est maintenue 10 heures à température ambiante. Après hydrolyse à 5°C par 20 ml d'une solution aqueuse saturée de chlorure d'ammonium, le DMF est évaporé et le résidu repris par 100 ml d'eau et extrait par 100 ml d'éther. Le produit attendu est obtenu après purification sur colonne de silice en utilisant comme éluant un mélange pentane/acétate d'éthyle 80/20.
Point de fusion : 68-70°C

| Microanalyse élémentaire: | | | |
|---|---|---|---|
| | C % | H % | N % |
| calculé | 68,44 | 8,16 | 4,20 |
| trouvé | 68,55 | 8,16 | 4,47 |

### Etude pharmacologique des dérivés de l'invention

### EXEMPLE 9: Activité in vivo sur les protéines de la phase aiguë

L'activité biologique des composés de l'invention a été déterminée en particulier sur l'albumine plasmatique du rat 6 jours après injection sous-cutanée d'adjuvant complet de Freund. Protéine négative de la phase aiguë de l'inflammation, l'albumine, fortement diminuée par l'état inflammatoire qui fait suite à l'adjuvant, est rétablie en tout ou partie par les composés administrés à la dose orale quotidienne de 100 mg/kg.

### Protocole expérimental

L'arthrite à l'adjuvant chez le rat, pour la première fois décrite par Pearson (Pearson CM., Proc. Soc. Exp. Biol. Med., 1956, 91, 95-101) a été provoquée par injection de 0,1 ml d'adjuvant complet de Freund (4 mg de mycobacterium butyricum en suspension dans 1 ml d'huile de paraffine/eau/tween 80) dans la région sous-plantaire de la patte postérieure du rat femelle Lewis (âgé de 62 jours). Les produits ont été administrés quotidiennement sous forme de solution aqueuse ou de suspension dans l'hydroxypropylcellulose à 0,2 % selon leur solubilité.

Leur activité sur les protéines de la phase aiguë a été évaluée par détermination des taux plasmatiques d'albumine 6 jours après l'induction de l'arthrite (méthode de dosage colorimétrique décrite par Lewis (Lewis EJ. & Coll., J. Pharmacol. Meth. 1989, 21, 183-94), l'adjuvant lui-même provoquant une chute de 31 % du taux d'albumine basal. L'atteinte clinique a été appréciée par mesure pléthysmométrique du volume de la patte postérieure infectée.

L'activité des composés de l'invention est très supérieure à celle d'un composé de référence : le romazarit, comme le montrent les résultats rassemblés ci-dessous :

| | correction de l'hypoalbuminémie |
|---|---|
| exemple 1 : 31 % | 31 % |
| exemple 2 : 47 % | 47 % |
| exemple 3 : 27 % | 27 % |
| romazarit : 1 % | 1 % |

Parallèlement, les composés tendent à diminuer l'intensité de l'oedème au site d'injection. C'est ainsi que le composé 2 diminue l'oedème de 9 %.

### EXEMPLE 10: Composition pharmaceutique

Formule de préparation pour 1000 comprimés dosés à 20 mg

| | |
|---|---|
| Composé de l'exemple 1 | 20 g |
| Hydroxypropylcellulose | 2 g |
| Amidon de blé | 10 g |
| Stéarate de magnésium | 100 g |
| Talc | 3 g |

## Revendications

1. Composés de formule (I) : 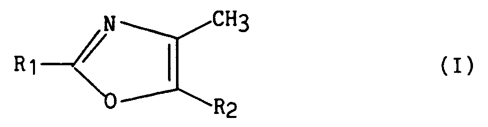 dans laquelle :
R₁ représente un groupement 1-adamantyl, dicyclopropylméthyle, cycloalkyle (C₃-C₆) (substitué ou non par un atome d'halogène, un groupement hydroxy, alkoxy (C₁-C₆) linéaire ou ramifié) ou bicyclo[2.2.2]oct-1-yl (substitué ou non en position 4 par un atome d'halogène, un groupement alkoxy (C₁-C₆) linéaire ou ramifié ou hydroxy),
R₂ représente un groupement
- 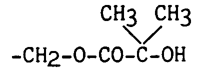 ou
- 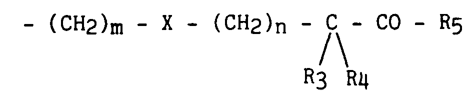
dans lequel :
m représente 1, 2 ou 3,
X représente un atome d'oxygène, de soufre ou un groupement N-R (dans lequel R est un atome d'hydrogène ou un groupement alkyle (C₁-C₆) linéaire ou ramifié),
R₃ ou R₄, identiques ou différents, représentent un atome d'hydrogène, un groupement alkyle (C₁-C₆) linéaire ou ramifié, trifluorométhyle,
ou 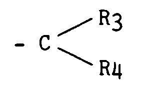 forme un radical cycloalkyle (C₃-C₆),
n représente 0, 1 ou 2,
R₅ représente un groupement hydroxy, alkoxy (C₁-C₆) linéaire ou ramifié, amino (substitué ou non par un ou deux groupements alkyle (C₁-C₆) linéaire ou ramifié),
- O - CH₂ - CO - NRR' (tel que R et R' représentent un groupement alkyle (C₁-C₆) linéaire ou ramifié, ou forment avec l'atome d'azote qui les portent un hétérocycle à 5 ou 6 chaînons),
leurs énantiomères, diastéréoisomères ou épimères ainsi que leurs sels d'addition à un acide ou à une base pharmaceutiquement acceptable.

2. Composés de formule (I) selon la revendication 1 tels que R₁ représente un groupement 1-adamantyl, leurs énantiomères, diastéréoisomères et épimères ainsi que leurs sels d'addition à un acide ou à une base pharmaceutiquement acceptable.

3. Composé de formule (I) selon la revendication 1 tels que R₁ représente un groupement dicyclopropylméthyl, ses énantiomères, diastéréoisomères et épimères ainsi que leurs sels d'addition à un acide ou à une base pharmaceutiquement acceptable.

4. Procédé de préparation caractérisé en ce que l'on utilise comme produit de départ un acide de formule (II) :
R₁ - CO₂H (II)
dans laquelle R₁ a la même signification que dans la formule (I), que l'on fait réagir sur le 2-chloroacétoacétate d'éthyle, pour conduire au composé de formule (III) : 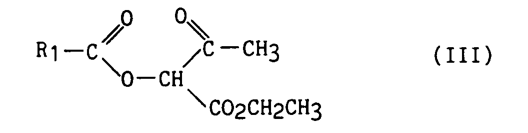 dans laquelle R₁ a la même signification que dans la formule (I),
qui subit l'action du formamide en milieu acide, pour conduire au composé de formule (IV) : 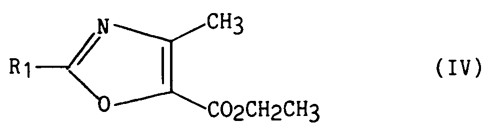 dans laquelle R₁ a la même signification que dans la formule (I),
que l'on transforme :
en composé de formule (V) par réduction en présence d'hydrure de lithium aluminium, 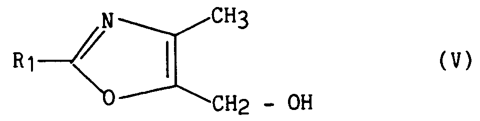 dans laquelle R₁ a la même signification que dans la formule (I), sur lequel on fait réagir :
soit,
en milieu anhydre, l'acétone en présence d'hydroxyde de sodium séché et de chloroforme,
pour conduire au composé de formule (I/a), cas particulier des composés de formule (I) 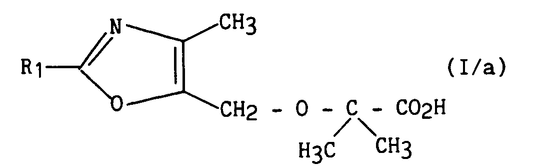 dans laquelle R₁ a la même signification que dans la formule (I), dont on transforme, si on le souhaite, la fonction acide en fonction ester ou amide selon des techniques classiques de la chimie organique,
soit,
le chlorure de thionyle, pour conduire au composé de formule (VI) : 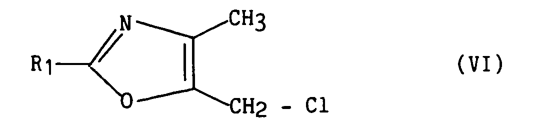 dans laquelle R₁ a la même signification que dans la formule (I), que l'on peut faire réagir, si on le souhaite, avec le 2-hydroxyisobutyrate d'éthyle, en présence d'hydrure de sodium en milieu diméthylformamide non anhydre, pour conduire au composé de formule (I/b) : 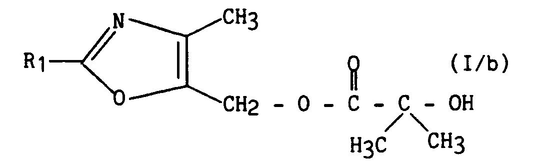 dans laquelle R₁ a la même signification que dans la formule (I),
composé de formule (VI) qui peut subir des réactions classiques de la chimie organique conduisant au composé de formule (VII) 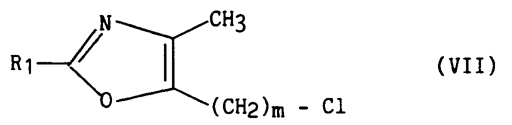 dans laquelle R₁ et m ont la même signification que dans la formule (I), que l'on fait réagir :
a avec un composé de formule (VIII), dans le DMF, en milieu anhydre : 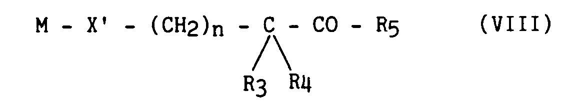 dans laquelle M représente un métal alcalin, n, R₃, R₄ et R₅ sont tels que définis dans la formule (I) et X' représente un atome de soufre ou d'oxygène,
pour conduire au composé de formule (I/c), cas particulier des composés de formule (I), 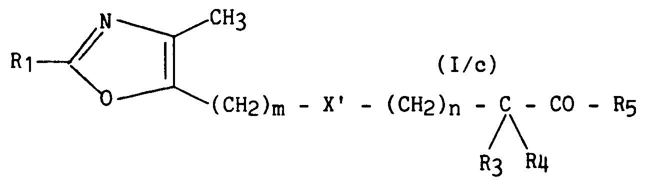 dans laquelle R₁, R₃, R₄, R₅, X', m et n sont tels que définis précédemment,
dont on transforme, si on le souhaite, R₅ lorsqu'il représente un groupement hydroxy en groupement amino ou ester correspondant selon les techniques classiques de la chimie organique,
b avec un amino-ester de formule (IX) : 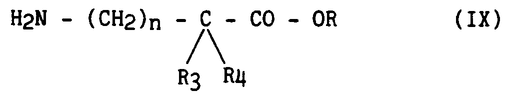 dans laquelle R représente un groupement alkyle, n, R₃ et R₄ ont la même signification que dans la formule (I), pour conduire au composé de formule( I/d), cas particulier des composés de formule (I) : 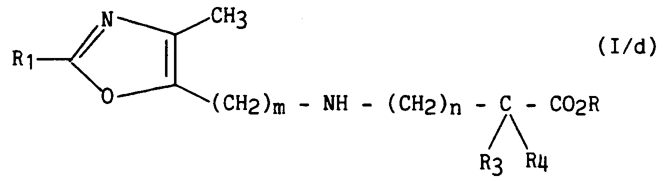 dans laquelle R₁, R₃, R₄, R, m et n ont la même signification que précédemment,
dont on transforme, si on le souhaite, la fonction ester en fonction acide puis en fonction amide correspondante selon des techniques classiques de la chimie organique et la fonction amine secondaire en fonction amine tertiaire par alkylation,
composés de formule (I/a), (I/b), (I/c) ou (I/d) que l'on purifie, le cas échéant, selon une technique classique de purification dont on sépare, si on le souhaite, les isomères selon des techniques classiques de séparation, et que l'on transforme éventuellement en leurs sels d'addition à un acide ou à une base pharmaceutiquement acceptable.

5. Compositions pharmaceutiques contenant comme principe actif au moins un composé selon l'une quelconque des revendications 1 à 3 seul ou en combinaison avec un ou plusieurs véhicules inertes non toxiques pharmaceutiquement acceptables.

6. Composition pharmaceutique selon la revendication 5 contenant au moins un principe actif selon l'une quelconque des revendications 1 à 3 utiles dans le traitement de l'arthrite et des maladies inflammatoires.
